# FASCICULE DE BREVET EUROPEEN

(11) **EP 1 372 617 B1**
(45) Date de publication et mention de la délivrance du brevet: **24.09.2008**
(21) Numéro de dépôt: 02757738.6
(22) Date de dépôt: 27.03.2002
(51) Int. Cl.: A61K 9/50, A61K 31/198, A61P 3/02, A61P 29/00

(54) **SELS DE CETOACIDES ET D'ACIDES AMINES GASTRORESISTANTS ET LEUR UTILISATION POUR LA PREPARATION DE MEDICAMENTS**
MAGENSAFTRESISTENTE SALZE VON KETOSÄUREN UND AMINOSÄUREN UND IHRE VERWENDUNG ZUR HERSTELLUNG VON MEDIKAMENTEN
GASTRORESISTANT SALTS OF KETOACIDS AND AMINO ACIDS AND THEIR USE FOR PREPARING MEDICINES

(30) Priorité: 29.03.2001 FR 0104259
(43) Date de publication de la demande: 02.01.2004
(73) Titulaire: CHIESI FARMACEUTICI S.p.A., 43100 Parma (IT)
(72) Inventeur: BOUYSSOU, Thierry, F-78650 Beynes (FR); BIOSA, Serge, F-78100 Mantes la Jolie (FR); JEANPETIT, Christian, F-78380 Bougival (FR); KILHOFFER, Daniel, F-27000 Evreux (FR); LE PEILLET-FEUILLET, Eliane, F-92100 Boulogne-Billancourt (FR)
(74) Mandataire: Minoja, Fabrizio
(86) Numéro de dépôt international: PCT/FR2002/001061
(87) Numéro de publication internationale: WO 2002/078676

(56) Documents cités:
- FR-A- 2 618 331
- FR-A- 2 775 901
- FR-A- 2 793 688
- US-A- 5 234 696

## Description

La présente invention a pour objet des sels de cétoacides et d'acides aminés gastrorésistants ainsi que leur utilisation pour la préparation de compositions pharmaceutiques destinées au traitement des sujets dénutris ou en situation d'hypercatabolisme ainsi qu'au traitement symptomatique des douleurs associées aux pathologies du tube digestif, de la vessie et des voies bilaires.

Les sels de cétoacides et d'acides aminés sont décrits dans le brevet WO 99/47134 comme étant actifs sur le modèle de distension du colon préalablement irrité à l'acide acétique 1 % chez le rat dès la doge de 1 mg/kg. Dans ce test, les composés sont administrés par voie orale, dissous dans l'eau.

L'α-cétoglutarate de di-onrnithine est également utilisé en thérapeutique sous le nom de CETORNAN (brevet français N°3 533 M) pour améliorer le métabolisme protéique des sujets dénutris. Il est également connu comme stimulant hormonal (hormone de croissance et insuline) et stimulant de la croissance cellulaire. La posologie à laquelle est utilisée ce composé est particulièrement élevée puisqu'il est administré en médecine de ville à la dose de 2 fois 5 g par jour par voie orale, dissous dans un verre d'eau et en médecine hospitalière à la dose de 2 fois 10 g par jour par voie entérale et orale, Cette posologie élevée pour ce type de composé a le désavantage de provoquer des diarrhées du fait d'une osmolalité importante (Pr L.CYNOBER, Dr P, CRENN, Pr B, MESSINE, La Revue du Praticien, N°50, 2000, pp 1593-1599). Un autre désavantage du produit est son mauvais goût en solution.

FR-A-2 618 331 décrit des compositions, gastroresistantes ou non, constituées par des mélanges de plusieurs sels formés par des α-cétoacides à chaîne ramifiée et des acides aminés basiques en tant que nutritionnels pour le traitement de l'insuffisance rénale chronique. De façon plus particulière, FR-A-2 618 331 divulgue des compositions contenant 3 cétoacides et 6 acides aminés, ou 5 cétoacides et 6 acides aminés, ou 5 cétoacides et 2 acides aminés. US-A-5 234 696 décrit un procédé de preparation de comprimés contenant une composition destinée aux traitement de l'insuffisance rénale chronique comprenant un mélange d'α-cétoacides à chaîne ramifiée et d'acides aminés. US-A-5 234 696 se rapporte à des compositions qui contiennent 4 cétoacides et trois acides aminés.

La présente invention découle de la mise en évidence par les inventeurs du fait totalement inattendu que les sels de cétoacides et d'acides aminés susmentionnés ne sont actifs dans le modèle de distension colique et dans un modèle de nutrition qu'à un pH neutre. Ces mêmes sels testés à pH 1 sont totalement inactifs, quelle que soit la dose testée.

Ainsi à titre d'illustration, le tableau I ci-après fait nettement apparaître que :
**➢** l'α-cétoglutarate de di-ornithine est actif à 1, 10 et 20 mg/kg p.o. à pH 6 et totalement inactif à pH 1 (tableau I(1)),
> l'α-cétoglutarate de mono-ornithine est actif à pH 6 et inactif à pH 1 (tableau I(2)).
> l'α-cétobutyrate d'arginine est actif à pH 6 et inactif à pH 1 (tableau I(3)).

Le tableau II ci-après fait apparaître que le mélange des constituants acide α-cétoglutarique et ornithine réalisé à pH 1 puis ramené à pH 6 est inactif (tableau II(1)).

De même, l'α-cétoglutarate de di-ornithine solubilisé à pH 1 entraînant une dissociation complète du sel, puis ramené à pH 6 est inactif. Une fois dissocié, l'α-cétoglutarate de di-ornithine ne se reconstitue pas. Seuls les constituants salifiés entre eux sont actifs (tableau II(2)).

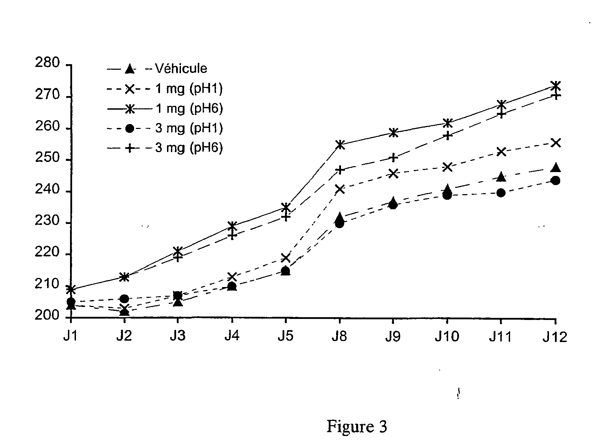

Le tableau VI fait apparaître que l'α-cétoglutarate de di-ornithine testé à 1 et 3 mg/kg dans un modèle de nutrition est actif à pH 6 et inactif à pH 1 (voir également figure 3).

Or, lorsqu'un sel de cétoacide et de dérivé aminé est administré par voie orale, celui-ci passe dans l'estomac à jeun où le pH est environ égal à 1. Par conséquent, il y a dissociation du sel en entités inactives et la posologie doit être augmentée pour qu'une partie du produit non dissociée passe la barrière stomacale et atteigne sa cible pharmacologique.

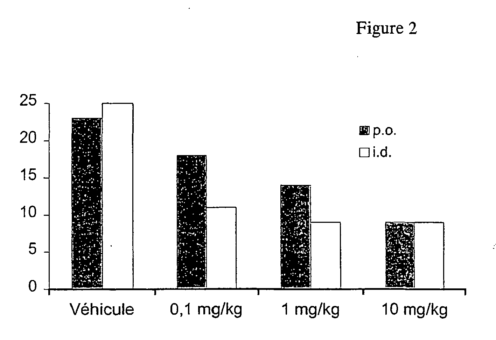

Le tableau V est à ce titre très représentatif de l'effet du pH. Ainsi, on peut noter que par voie intraduodénale (pH neutre) qui évite l'acidité gastrique, l'α-cétoglutarate de di-ornithine est actif dès la dose de 0,1 mg/kg soit une dose dix fois inférieure à la dose minimum active par voie orale (tableau V(2) et figure 2).

La présente invention a pour objet des compositions gastrorésistantes comprenant des composés de formule générale (I) suivante :

(X)ₙ₁,Y,(Z)ₙ₂ (I)

dans laquelle :
- X et Z sont des acides aminés identiques ou différents, naturels ou non,
- Y est un cétoacide à chaîne linéaire non ramifiée,
- n₁ et n₂, indépendamment l'un de l'autre, représentent 0 ou 1, l'un au moins de n₁, et n₂ représentant 1, ledit composé se présentant sous forme de sel entre deux constituants X et Y, ou Y et Z, ou entre les trois constituants X, Y et Z.

Lesdits composés au sein desdites compositions, sont stables en milieu neutre et à pH inférieur à 6. Avantageusement, les composés au sein des dites compositions sont stables jusqu' à un pH d' environ 1.

Il est bien entendu que les composés de formule (I) susmentionnée résultent de la formation de liaisons ioniques principalement, et en aucun cas de liaisons covalentes, entre les différents constituants X, Y ou Z. Par conséquent, l'ordre d'apparition de ces différents constituants dans la formule (I) n'a pas de signification particulière, et cette formule (I) doit être comprise comme comprenant aussi bien les composés de formule (X)ₙ₁,Y,(Z)ₙ₂, que ceux de formule (Z)ₙ₂,Y,(X)ₙ₁; (X)ₙ₁,(Z)ₙ₂,Y ;(Z)ₙ₂,(X)ₙ₁,Y ; Y,(X)ₙ₁,(Z)ₙ₂ ; Y,(Z)ₙ₂,(X)ₙ₁.

La résistance au milieu acide permet de conserver l'activité par voie orale. A titre d'exemple, le tableau III fait nettement apparaître que la forme gastrorésistante de l'α-cétoglutarate de di-ornithine est active à 1 mg/kg. Cette activité est équivalente à la dose de 0,1 mg/kg par voie intraduodénale, et bien supérieure aux microgranules non gastrorésistantes ou au sel administré en solution. Le tableau IV montre bien la supériorité de la forme gastrorésistante puisque celle-ci s'avère active dès 0,25 mg/kg dans un modèle de douleur viscérale.

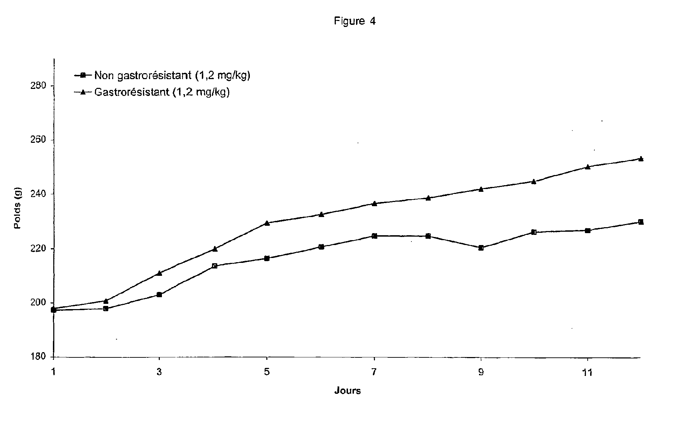

Le tableau VII complète la démonstration de l'effet pH. Il fait apparaître l'activité de l'α-cétoglutarate de di-ornithine en composition gastrorésistante versus composition non gastrorésistante dans une étude de bioéquivalence à 1,2 mg / kg sur un modèle de nutrition (voir également figure 4).

Un deuxième avantage de l'invention est la diminution des diarrhées induites par les composés du fait de la gastrorésistance, de la diminution de la posologie, et par conséquent de l'osmolalité.

Le tableau VIII fait apparaître qu'une composition gastrorésistante versus une composition non gastrorésistante d'α-cétoglutarate de di-ornithine entraîne une diminution très significative des diarrhées dans un modèle de nutrition.

Un autre avantage est le masquage du mauvais goût de par l'enrobage de la composition.

Avantageusement, les compositions gastrorésistantes de l'invention sont telles que les composés sous forme de sels contenus dans ces compositions ne se dissocient pas dans l'estomac, en produits de dissociation, ou se dissocient en produits de dissociation susmentionnés dans des proportions inférieures à environ 20 % , et de préférence inférieures à environ 10%, ou encore inférieures à 20 % à pH 1.

Plus particulièrement, les composés susmentionnés des compositions gastrorésistantes de l'invention ne se dégradent pas en produits de dissociation dans un intervalle de pH d'environ 7 à environ 1.

Par produits de dissociation susmentionnés, on entend les molécules restantes après scission d'une ou plusieurs des liaisons d'un composé susmentionné, notamment :
- les produits correspondant d'une part au sel formé entre le cétoacide Y et un seul des deux acides aminés X ou Z, et, d'autre part, l'autre acide aminé X ou Z non engagé dans le sel susmentionné,
- ou les produits correspondant d'une part au cétoacide Y, et, d'autre part, aux acides aminés X et/ou Z, non engagés dans un sel avec Y.

A titre d'illustration, les produits de dissociation de l'α-cétoglutarate de di-ornithine, sont l'α-cétoglutarate d'ornithine et/ou l'acide α-cétoglutarique et l'ornithine.

La présente invention a plus particulièrement pour objet les compositions gastrorésistantes telles que définies ci-dessus, comprenant des composés de formule générale (I) dans laquelle :
- n₁ et n₂, indépendamment l'un de l'autre, représentent 0 ou 1, l'un au moins de n₁ et n₂ représentant 1,
- X représente un aminoacide naturel, sous réserve que lorsque n₂ = 0 alors X représente un aminoacide basique tel que :
   - l'ornithine,
   - l'arginine,
   - la lysine,
   - ou, l'histidine,
- Y représente un cétoacide de formule (II) suivante :

   R-CO-COOH (II)

   dans laquelle R représente un groupe alkyle ou acide alcanoïque linéaire, d'environ 1 à environ 10 atomes de carbone, notamment un cétoacide de formule (II) dans laquelle lorsque R représente :
   - -CH₃, ledit cétoacide est l'acide pyruvique,
   - -CH₂-CH₃, ledit cétoacide est l'acide α-cétobutyrique,
   - -(CH₂)₂-COOH, ledit cétoacide est l'acide α-cétoglutarique,
   - -(CH₂)₃-COOH, ledit cétoacide est l'acide α-cétoadipique,
- Z représente un aminoacide naturel, notamment un aminoacide choisi parmi l'ornithine, l'arginine, la lysine, l'histidine, la proline, ou la glutamine.

L'invention a plus particulièrement pour objet les compositions gastrorésistantes susmentionnées comprenant des composés de formule (I) telle que définie ci-dessus, dans laquelle Y représente un cétoacide choisi parmi l'acide α-cétoglutarique, ou l'acide α-cétobutyrique.

L'invention concerne plus particulièrement les compositions gastrorésistantes susmentionnées, caractérisées en ce que les composés sont choisis parmi ceux de formule (I) dans laquelle :
- n₁=1, et n₂ = 0 ou 1,
- X représente un acide aminé choisi parmi l'ornithine, la lysine, ou l'arginine,
- Y représente un cétoacide choisi parmi l'acide α-cétoglutarique, ou l'acide α-cétobutyrique,
- et, lorsque n₂ = 1, Z représente un acide aminé naturel, notamment l'ornithine, l'arginine, la proline, ou la glutamine.

L'invention a plus particulièrement pour objet les compositions gastrorésistantes susmentionnées, caractérisées en ce que les composés sont choisis parmi ceux de formule (I) dans laquelle :
- n₁=1, et n₂=0,
- X représente l'ornithine et Y représente l'acide α-cétoglutarique, à savoir l'α-cétoglutarate de mono-ornithine,
- X représente l'ornithine et Y représente l'acide α-cétobutyrique, à savoir l'α-cétobutyrate de mono-ornithine,
- X représente l'arginine et Y représente l'acide α-cétobutyrique, à savoir l'α-cétobutyrate d'arginine,
- X représente la lysine et Y représente l'acide α-cétobutyrique, à savoir l'α-cétobutyrate de lysine,
- X représente l'histidine et Y représente l'acide α-cétobutyrique, à savoir l'α-cétobutyrate d'histidine.

L'invention concerne plus particulièrement encore les compositions gastrorésistantes susmentionnées, caractérisées en ce que les composés sont choisis parmi ceux de formule (I) dans laquelle :
- n₁=1, et n₂=1,
- X représente l'ornithine, Y représente l'acide α-cétoglutarique, et Z représente l'ornithine, à savoir l'α-cétoglutarate de di-ornithine,
- X représente l'arginine, Y représente l'acide α-cétoglutarique, et Z représente l'arginine, à savoir l'α-cétoglutarate de di-arginine,
- X représente l'ornithine, Y représente l'acide α-cétoglutarique, et Z représente la glutamine, à savoir l'α-cétoglutarate d'ornithine et de glutamine,
- X représente l'ornithine, Y représente l'acide α-cétoglutarique, et Z représente la proline, à savoir l'α-cétoglutarate d'ornithine et de proline.

De préférence, le composé de formule (I) compris dans les compositions gastrorésistantes susmentionnées dans le cadre de la présente invention, est l'α-cétoglutarate de di-ornithine.

L'invention a également pour objet les compositions gastrorésistantes susmentionnées comprenant, à titre de composés de formule (I) préférés, ceux choisis dans le groupe constitué par l'α-cétobutyrate d'arginine, l'α-cétobutyrate de lysine, l'α-cétobutyrate d'histidine.

L'invention a plus particulièrement pour objet les compositions gastrorésistantes susmentionnées, comprenant des composés de formule (I) dans laquelle :
- n₁=0 ou 1, et n₂= 1,
- lorsque n₁ = 1, X représente un aminoacide naturel, tel que l'ornithine, ou l'arginine,
- Y représente un cétoacide choisi parmi l'acide α-cétoglutarique, ou l'acide α-cétobutyrique.

Avantageusement, les composés tels que décrits ci-dessus se présentent sous forme de sels entre deux constituants Y et Y, ou Y et Z, ou entre les trois constituants X, Y et Z.

La proportion en poids des différents constituants X, Y et/ou Z est de préférence comprise entre 0,8 et 1,2, de sorte que la somme des proportions de chacun des constituants est égale à 2 dans un sel entre deux constituants X et Y, ou Y et Z, ou est égale à 3 dans un sel entre trois constituants X, Y et Z.

Avantageusement, la proportion susmentionnée des différents constituants est comprise entre 0,9 et 1,1.

Des composés particulièrement préférés sont ceux au sein desquels les différents constituants X et Y, ou Y et Z, ou X,Y et Z sont dans un rapport équimolaire, c'est-à-dire que chacun des constituants est dans une proportion en poids de 1, de sorte que la somme des proportions de chacun des constituants est égale à 2 dans un sel entre deux constituants X et Y, ou Y et Z, ou est égale à 3 dans un sel entre les trois constituants X,Y et Z.

L'invention a également pour objet les compositions gastrorésistantes susmentionnées, caractérisées en ce que le véhicule gastrorésistant physiologiquement stable est choisi parmi des formes galéniques gastrorésistantes choisies à titre d'exemple parmi les suivantes :
- les microgranules gastrorésistantes, et plus particulièrement celles choisies parmi les supports neutres à base de saccharose et d'amidon de maïs ou autre,
- les microgranules pelliculées gastrorésistantes, et plus particulièrement celles choisies parmi les microgranules définies précédemment sur lesquelles a été pulvérisée une solution d'agent filmogène et/ou plastifiant,
- les nanoparticules gastrorésistantes, et plus particulièrement celles choisies parmi les capsules constituées d'un matériel polymérisé capable de retenir les principes actifs par séquestration ou adsorption de taille comprise entre 50 et 300 nm, ou les nanosphères gastrorésistantes, et plus particulièrement celles choisies parmi les nanoparticules susmentionnées de forme sphérique,
- les microsphères gastrorésistantes, et plus particulièrement celles choisies parmi les produits solides pleins et sphériques,
- les microcapsules gastrorésistantes, et plus particulièrement celles choisies parmi les produits solides constitués d'une enveloppe elle-même solide contenant un liquide, un solide
ou une substance pâteuse,
- les sphéroïdes et les sphérules gastrorésistants de produits solides pleins avec ou sans excipients,
- les granulés gastrorésistants, et plus particulièrement ceux choisis parmi les produits solides obtenus à partir de saccharose et/ou lactose par exemple,
- les sphéroïdes et les sphérules pelliculés gastrorésistants, de produits solides pleins avec ou sans excipients obtenus par extrusion et/ou sphéronisation, par exemple sur lesquels a été pulvérisée une solution d'agent filmogène et/ou plastifiant et autre excipient de quelque nature que ce soit, garantissant la qualité du film,
- les granulés pelliculés gastrorésistants, et plus particulièrement ceux choisis parmi les granulés définis précédemment sur lesquels a été pulvérisée une solution d'agent filmogène et/ou plastifiant et autre excipient de quelque nature que ce soit, garantissant la qualité du film,
- les liposomes gastrorésistants, et plus particulièrement ceux choisis parmi les vésicules sphériques dont le centre est occupé par une cavité aqueuse et l'enveloppe constituée de feuillets à base de phospholipides dont la taille est de préférence inférieure ou égale à 1µm,
- les liposomes pelliculés gastrorésistants, et plus particulièrement ceux choisis parmi les liposomes définis précédemment sur lesquels a été pulvérisée une solution d'agent filmogène et/ou plastifiant et autre excipient de quelque nature que ce soit, garantissant la qualité du film,
- les lyocs gastrorésistants, et plus particulièrement ceux choisis parmi les produits obtenus par lyophilisation de dispersions de vésicules inférieures ou égales à 1 µm,
- les lyocs pelliculés gastrorésistants, et plus particulièrement ceux choisis parmi les lyocs définis précédemment sur lesquels a été pulvérisée une solution d'agent filmogène et/ou plastifiant et autre excipient de quelque nature que ce soit, garantissant la qualité du film,
- les pompes osmotiques dans un enrobage gastrorésistant, et plus particulièrement ceux choisis parmi les comprimés enrobés ordinaires constitués d'un noyau osmotiquement actif, d'une membrane semi-perméable et d'un orifice calibré dans la membrane,
- les gommes, telles que gomme sterculia, Adragante, xanthane, arabique...,
- les comprimés pelliculés gastrorésistants de produits solides pleins avec ou sans excipient sur lesquels a été pulvérisée une solution d'agent filmogène et/ou plastifiant et autre excipient de quelque nature que ce soit, garantissant la qualité du film,
- les gélules pelliculées gastrorésistantes de produits solides pleins avec ou sans excipient sur lesquels a été pulvérisée une solution d'agent filmogène et/ou plastifiant et autre excipient de quelque nature que ce soit, garantissant la qualité du film.

Avantageusement, les compositions susmentionnées peuvent se présenter sous forme de comprimés, de gélules, de sachets, ou de granulés, gastrorésistants, tels que décrits ci-dessus,
ou toutes autres formes et conditionnements appropriés.

Ces différentes formes peuvent être obtenues selon les méthodes décrites dans "Coated Pharmaceutical Dosage Forms", de Bauer, Lehmann, Osterwald et Rothgang, édité par Medpharm Scientific Publishers et dans "Pharmacotechnie Industrielle" de Yves Rossetto.

L'invention a également pour objet les adjuvants de nutrition caractérisés en ce qu'ils contiennent une composition gastrorésistante telle que définie ci-dessus, le cas échéant en association avec un véhicule acceptable en alimentation.

Avantageusement, les adjuvants de nutrition susmentionnés se présentent sous une forme prête à diluer ou à disperser dans un solvant aqueux.

De préférence, les adjuvants de nutrition susmentionnés se présentent sous forme de prise unitaire contenant environ 10 ou 25 mg jusqu'à environ 20 g ou 50 g d'au moins un composé de formule (I) défini ci-dessus.

L'invention a plus particulièrement pour objet l'utilisation d'un adjuvant de nutrition tel que défini ci-dessus, dans le cadre du traitement des sujets en état de dénutrition, notamment :
- des sujets dénutris,
- des sujets anorexiques,
- des patients atteints de la gastroparésie,
- des sujets dont le transit digestif est ralenti,
- des sujets souffrant de malabsorption digestive,
- des sujets dialysés,
- des patients atteints de la maladie d'Alzheimer,
- des personnes en situation d'hypercatabolisme, tels que
   - des personnes atteintes d'insuffisance respiratoire,
   - les personnes souffrant d'escarres,
   - les patients brûlés, ,
   - les patients cancéreux,
   - les patients atteints du SIDA,
   - les patients post-opérés,
   - les personnes en cours de cicatrisation de la muqueuse intestinale,
   - les patients polytraumatisés,
   - les patients insuffisants cardiaques,
   ou dans le cadre de la cicatrisation et de la stimulation de l'hormone de croissance, ou de l'insuline.

L'invention a plus particulièrement pour objet l'utilisation susmentionnée d'un adjuvant de nutrition tel que défini ci-dessus, à raison d'une posologie journalière comprise entre environ 50 mg à environ 40 g.

L'invention a également pour objet toute composition pharmaceutique caractérisée en ce qu'elle contient une composition gastrorésistante telle que définie ci-dessus, le cas échéant en association avec un véhicule pharmaceutiquement acceptable.

Avantageusement, les compositions pharmaceutiques susmentionnées se présentent sous une forme administrable par voie orale, ou par voie entérale, notamment sous une forme sèche à diluer ou à disperser dans un solvant aqueux lorsqu'elle est sous une forme administrable par voie orale. Suivant le patient et le type et la sévérité de la maladie à traiter, le dosage classique des compositions pharmaceutiques est susceptible de varier entre environ 10 mg et environ 40 g d'ingrédient actif.

De préférence, les compositions pharmaceutiques susmentionnées sont administrables par voie orale et entérale, en dose unitaire de 25 mg à 10 g de principe actif (composé de formule (I)) par prise, et de préférence de 100 mg à 5 g, à raison de 1 à 2 prises par jour.

L'invention concerne également l'utilisation d'une composition gastrorésistante telle que définie ci-dessus, pour la préparation d'un médicament destiné au traitement des sujets dénutris susmentionnés, ou des animaux.

L'invention a également pour objet l'utilisation d'une composition gastrorésistante telle que définie ci-dessus, pour la préparation d'un médicament destiné au traitement des états de dénutrition susmentionnés, ou de pathologies humaines ou animales dans lesquelles sont impliqués les neurones silencieux, telles que les pathologies du tube digestif, de la vessie et des voies biliaires, et plus particulièrement au traitement symptomatique des douleurs associées à ces pathologies, telles que les douleurs :
- des troubles du transit et de l'inconfort intestinal liés aux troubles fonctionnels intestinaux (dyspepsies, syndrome de l'intestin irritable, côlon irritable, ...),
- des voies biliaires,
- dans la rectocolite hémorragique,
- dans la maladie de Crohn,
- dans l'ulcère gastrique et duodénal,
- dans la gastrite chronique,
- dans le cancer colorectal ou gastrique,
- dans la gastroentérite et la grippe intestinale,
- liées à une pseudo-obstruction intestinale ou colique,
- dans l'iléite radique,
- post-opératoires digestives ou viscérales,
- dans la diarrhée, spasmes, constipations, mégacôlon, le mégarectum,
- des spasmes de la vessie,
- de la parésie vésicale.

L'invention a plus particulièrement pour objet l'utilisation susmentionnée de compositions gastrorésistantes telles que définies ci-dessus, pour la préparation d'un médicament susceptible d'être administré à raison d'une posologie journalière en principe actif comprise entre environ 1 mg/kg/jour à 1 g/kg/jour, de préférence 20 à 200 mg/kg/jour par voies orale et entérale.

L'invention concerne également l'utilisation de compositions gastrorésistantes comprenant au moins un composé de formule générale (Ibis) suivante :

(X)ₙ₁,Y,(Z)ₙ₂ (Ibis)

dans laquelle :
- X et Z sont des acides aminés identiques ou différents, naturels ou non,
- Y est un cétoacide à chaîne ramifiée,
- n₁ et n₂, indépendamment l'un de l'autre, représentent 0 ou 1, l'un au moins de n₁ et n₂ représentant 1, ledit composé se présentant sous forme de sel entre deux constituants X et Y, ou Y et Z, ou entre les trois constituants X, Y et Z,
   pour la préparation d'un adjuvant de nutrition, ou d'un médicament, destinés au traitement des états de dénutrition.

L'invention a plus particulièrement pour objet l'utilisation de compositions gastrorésistantes susmentionnées comprenant au moins un composé de formule (Ibis), pour la préparation d'un adjuvant de nutrition ou d'un médicament destiné au traitement des états de dénutrition, notamment chez les sujets susmentionnés, ou dans le cadre de la cicatrisation et de la stimulation de l'hormone de croissance, ou de l'insuline.

L'invention concerne plus particulièrement l'utilisation de compositions gastrorésistantes susmentionnées comprenant an moins un composé de formule (Ibis), pour la préparation d'un médicament destiné au traitement symptomatique des douleurs associées aux pathologies du tube digestif, de la vessie et des voies biliaires.

L'invention a plus particulièrement pour objet l'utilisation de compositions gastrorésistantes susmentionnées comprenant au moins un composé de formule (Ibis), pour la préparation d'un médicament destiné au traitement symptomatique des douleurs listée ci-dessus.

L'invention concerne plus particulièrement l'utilisation de compositions gastrorésistantes susmentionnées comprenant au moins un composé de formule (Ibis) dans laquelle :
- n₁ et n₂, indépendamment l'un de l'autre, représentent 0 ou 1, l'un an moins de n₁ et n₂ représentant 1,
- X représente un aminoacide naturel, sous réserve que lorsque n₂ = 0 alors X représente un aminoacide basique tel que :
   - l'arginine,
   - la lysine,
   - ou, l'histidine,
- Y représente un cétoacide de formule (II) suivante :

   R-CO-COOH (II)

   dans laquelle R représente un groupe alkyle ou acide alcanoïque ramifié, d'environ 1 à environ 10 atomes de carbone, notamment un cétoacide de formule (II) dans laquelle lorsque R représente:
   - -CH(CH₃)₂, ledit cétoacide est l'acide α-cétoisovalérique,
   - -CH(CH₃)-CH₂-CH₃, ledit cétoacide est l'acide α-céto β-méthylvalérique,
   - -CH₂-CH(CH₃)₂, ledit cétoacide est l'acide α-cétoisocaproïque.

L'invention a plus particulièrement pour objet l'utilisation de compositions gastrorésistantes susmentionnées, comprenant au moins un composé de formule générale (Ibis) dans laquelle :
- X représente l'arginine et Y représente l'acide α-céto-isocaproïque, à savoir l'α-céto-isocaproate d'arginine,
- X représente l'ornithine et Y représente l'acide α-céto-isocaproïque, à savoir l'α-céto-isocaproate d'ornithine,
- X représente l'ornithine et Y représente l'acide α-céto-β méthylvalérique, à savoir l'α-céto-β méthylvalérate d'ornithine,
- X représente l'arginine et Y représente l'acide α-céto-β méthylvalérique, à savoir l'α-céto-β méthylvalérate d'arginine,
- X représente l'arginine et Y représente l'acide α-céto-isovalérique, à savoir l'α-céto-isovalérate d'arginine,
- X représente l'ornithine et Y représente l'acide α-céto-isovalérique, à savoir l'α-céto-isovalérate d'ornithine.

L'invention sera davantage illustrée à l'aide de la description détaillée qui suit de la préparation des compositions gastrorésistantes de l'invention et de leur propriétés analgésiques mesurées sur un modèle de distension colique ainsi que de leur propriétés en tant qu'adjuvant de nutrition mesuré sur un modèle d'hypercatabolisme protéique.

### 1 - Procédé de préparation

### 1- Matériel utilisé

Lit d'air fluidisé Aéromatic^{®} de type STREA 1
Pompe péristaltique de type Bioblock^{®}
Etuve Memmert^{®}
Buse 0,8 mm d'ouverture

### 2 - Matières utilisées

| | |
|---|---|
| Identité | Fournisseur |
| Microgranules T16-18 | SPCI/Mendell |
| PVP K30 | BASF |
| Eudragit L30D-55 | Rohm Pharma |
| Triéthyl Citrate (TEC) | SPCI |
| Eau bi-osmosée | |

### 3 - Montage

Une solution aqueuse (92,4 ml) contenant l'α-cétoglutarate de di-ornithine (4,80 g) et de la PVPK30 (2,89 g) est pulvérisée sur des microgranules (300,1 g) préchauffées 10 minutes à une température de 30 à 35°C, durant environ 113 minutes, à un débit de 1 ml/min sous une pression de 1 bar.
La ventilation est réglée à 4 au début puis augmentée à 5, la température d'entrée étant de 43°C et la température de sortie de 37°C, on obtient 303,37 g de microgranules montées.

### 4 - Pelliculage

### Le pelliculage a été réalisée en 3 phases :

a) 1^{ére} phase
   Une solution aqueuse (115 ml) contenant 268,6 g d'Eudragit et 8 g de Triéthyl citrate est pulvérisée sur 200 g de microgranules préalablement obtenues durant 189 minutes à un débit de 1,6 ml/min, sous une pression de 1,1 bars. La ventilation est réglée à 4,5 au début puis augmentée à 5, la température d'entrée est de 35°C et la température de sortie de 30°C.
   On obtient 220 g de microgranules.
b) 2^{éme} phase
   Une solution aqueuse (247,8 ml) contenant 462,9 g d'Eudragit, 14,5 g de Triéthyl citrate est pulvérisée sur 200 g de microgranules obtenues à la phase 1 durant 168 minutes, à un débit compris entre 1,8 et 2,6 ml/min sous une pression de 0,8 bar. La ventilation est réglée à 3,5, la température d'entrée est de 36°C, la température de sortie est de 28°C.
   On obtient 236 g de microgranules.
c) 3^{ème} phase
   Une solution aqueuse identique à la phase 2 est pulvérisée sur 200 g de microgranules obtenues à la phase 2 durant 148 minutes dans les mêmes conditions que précédemment hormis le débit qui est de 2,6 ml/min.
   On obtient 238 g de microgranules pelliculées qui contiennent chacune 6,4 µg d'α-cétoglutarate de di-ornithine, 3,86 µg de PVP, 469,81 µg d'Eudragit et 48,62 µg de Triéthyl citrate.

### II - Pharmacologie

### Distension colique

L'activité analgésique a été étudiée sur un modèle de douleur digestive chez le rat vigile. Cette douleur est provoquée par la distension du côlon à l'aide d'un ballonnet.

### Protocole

Les rats mâles Sprague-Dawley d'environ 180 g à jeun depuis la veille sont utilisés. Sous légère anesthésie au fluothane, une sonde intrarectale est introduite à 5 cm de l'anus et 1,5 ml d'acide acétique à 1 % est injecté. Une heure trente après l'irritation, un ballonnet en latex (∅ à vide 2 mm, longueur 1 cm) monté sur un cathéter en polyéthylène est introduit dans le côlon sur le site de l'irritation.
Le produit à tester ou le véhicule (eau distillée) est administré per os sous un volume de 1 ml, puis le rat est mis en observation dans un cristallisoir.
La distension du côlon est réalisée 2h30 après l'irritation. Elle est effectuée sous un volume fixe égal à 1,5 ml d'eau distillée. La distension colique provoque une douleur digestive objectivée par des crampes abdominales dont le nombre reflète l'intensité de la douleur. La distension colique est maintenue pendant 10 minutes au cours desquelles les crampes abdominales sont dénombrées.
L'analyse statistique est effectuée à l'aide du test de Dunnett qui compare un même groupe d'animaux véhicule (40) à plusieurs groupes de rats (6 animaux par groupe) ayant reçu les molécules étudiées. Le seuil de signification est fixé à 5 % .
Les molécules sont testées par voie orale à 1-10-20 mg/kg. Elles sont solubilisées dans de l'eau distillée.
Le véhicule utilisé comme placebo se compose d'eau distillée.

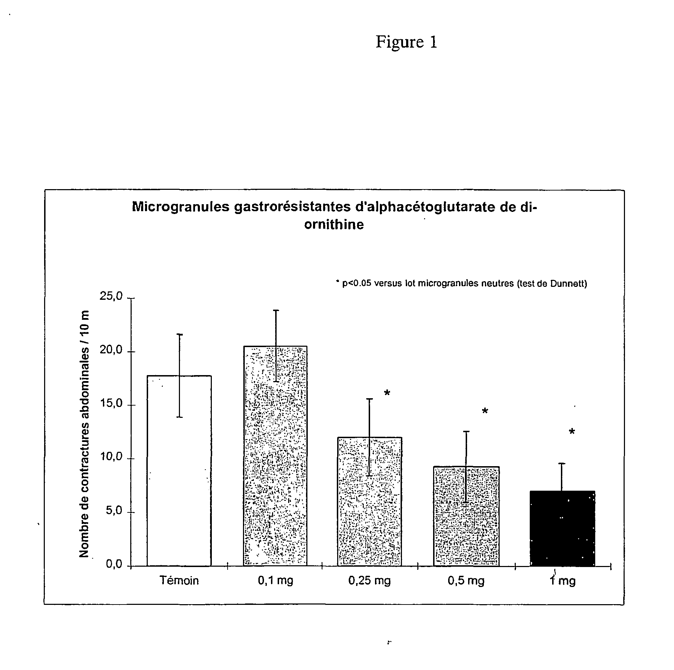

Résultats : tableaux I, II, III, IV, et V, et figures 1 à 2

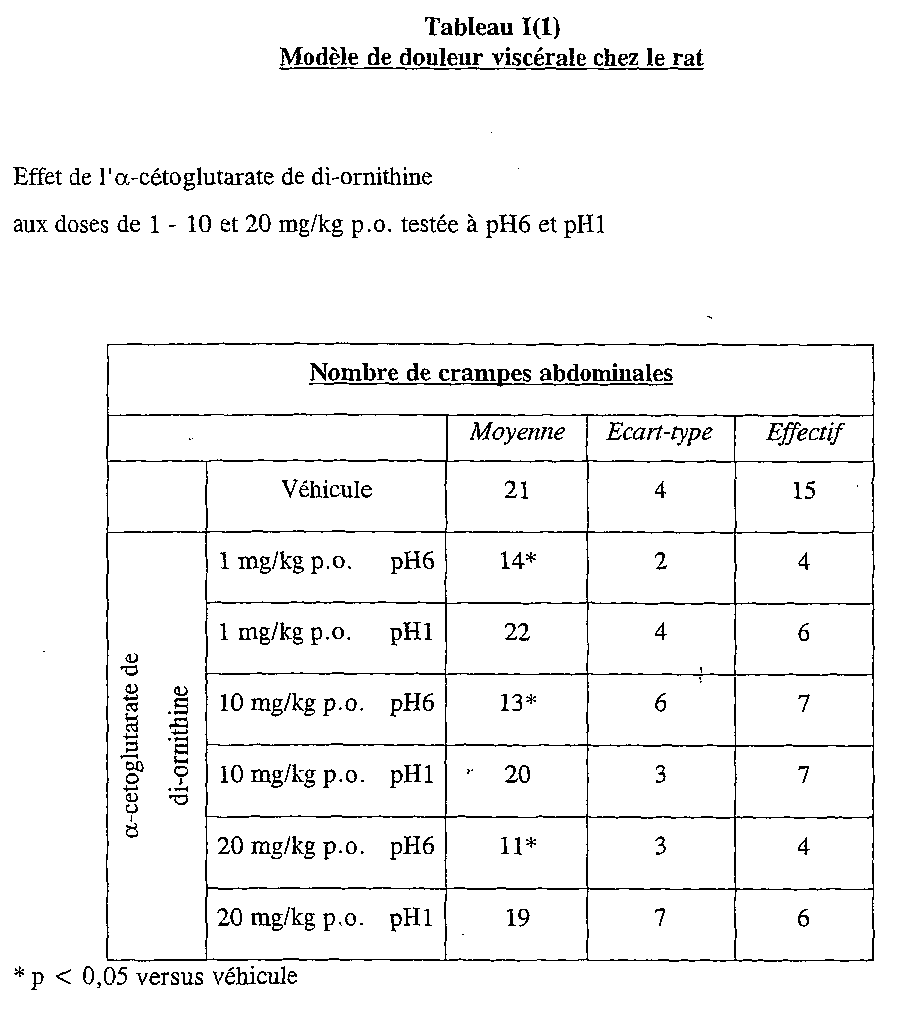

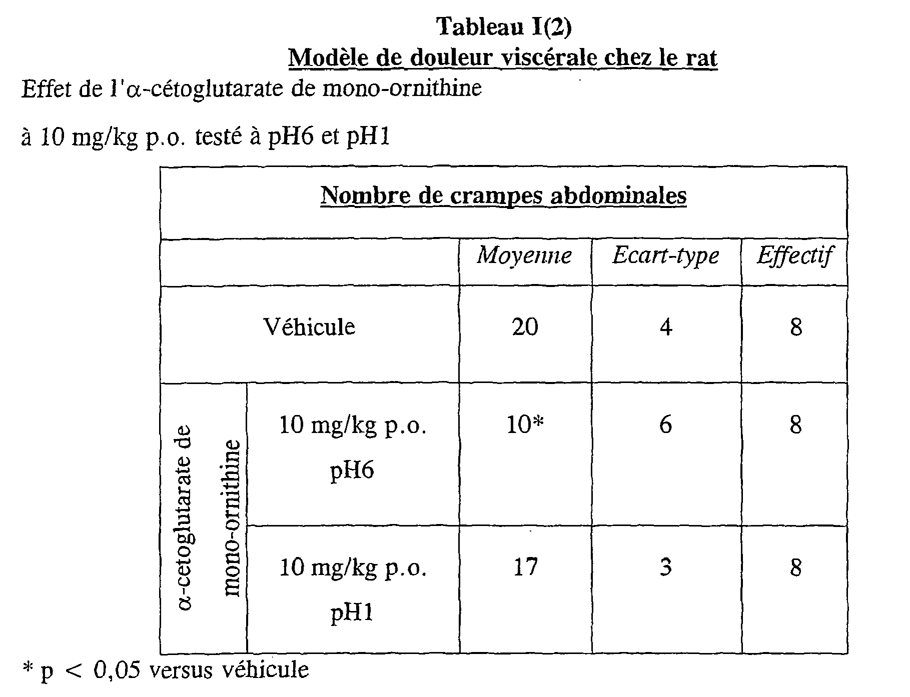

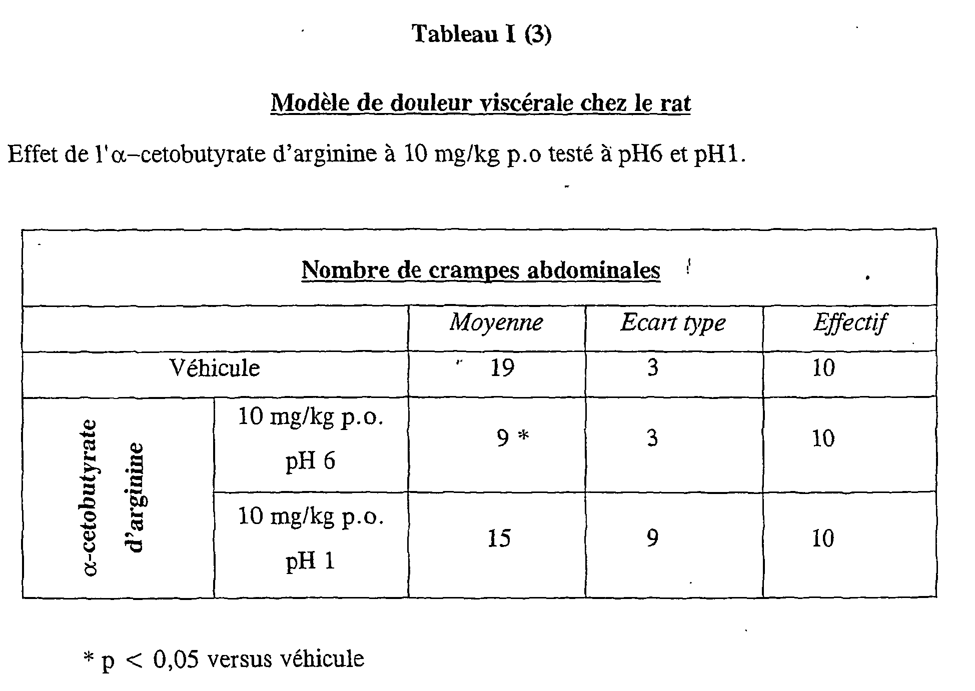

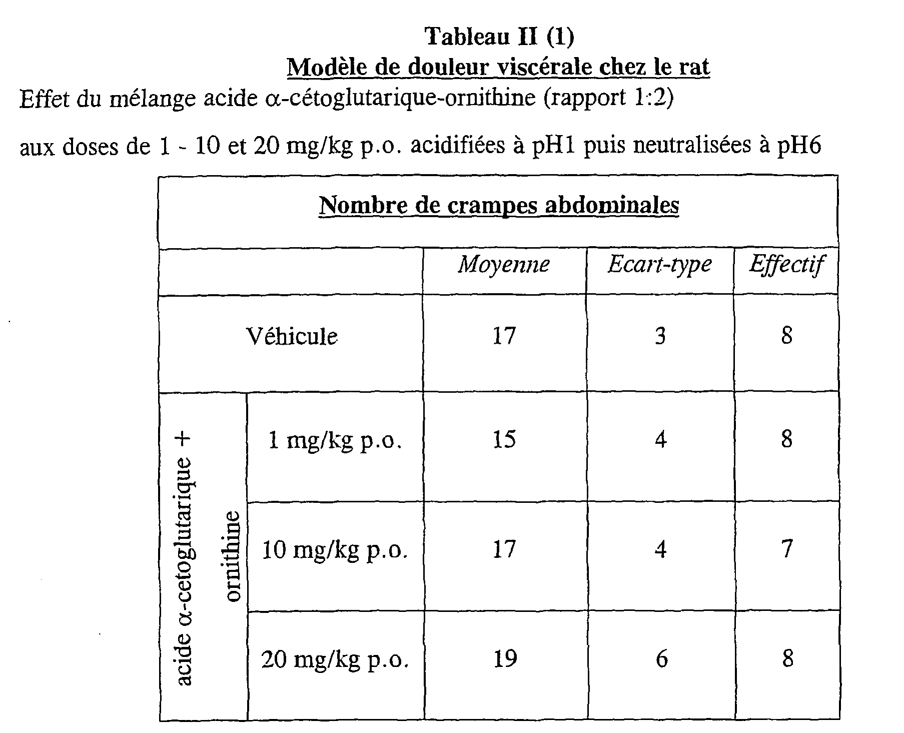

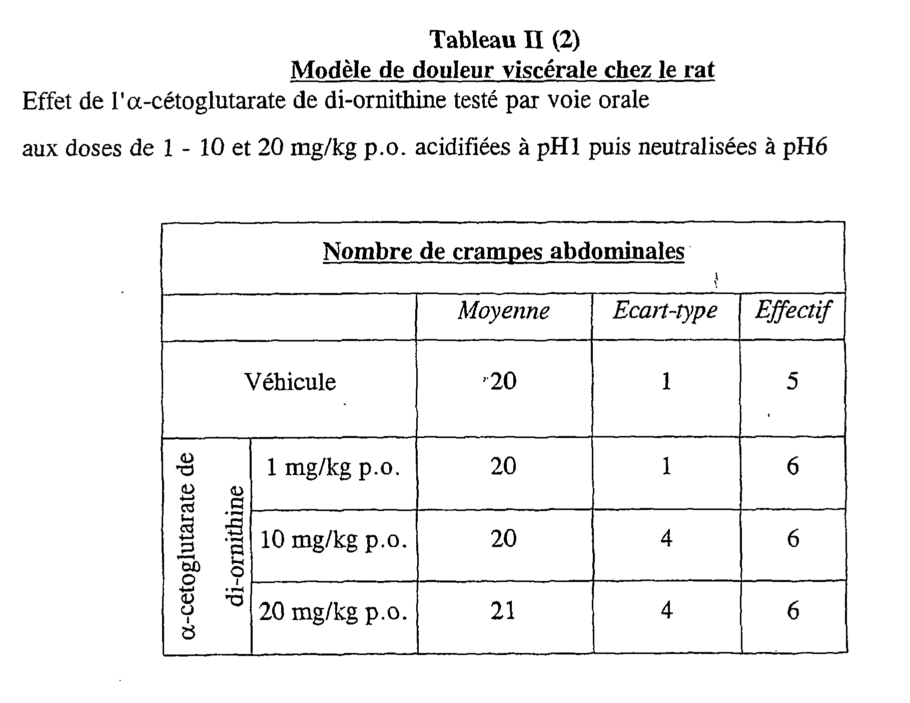

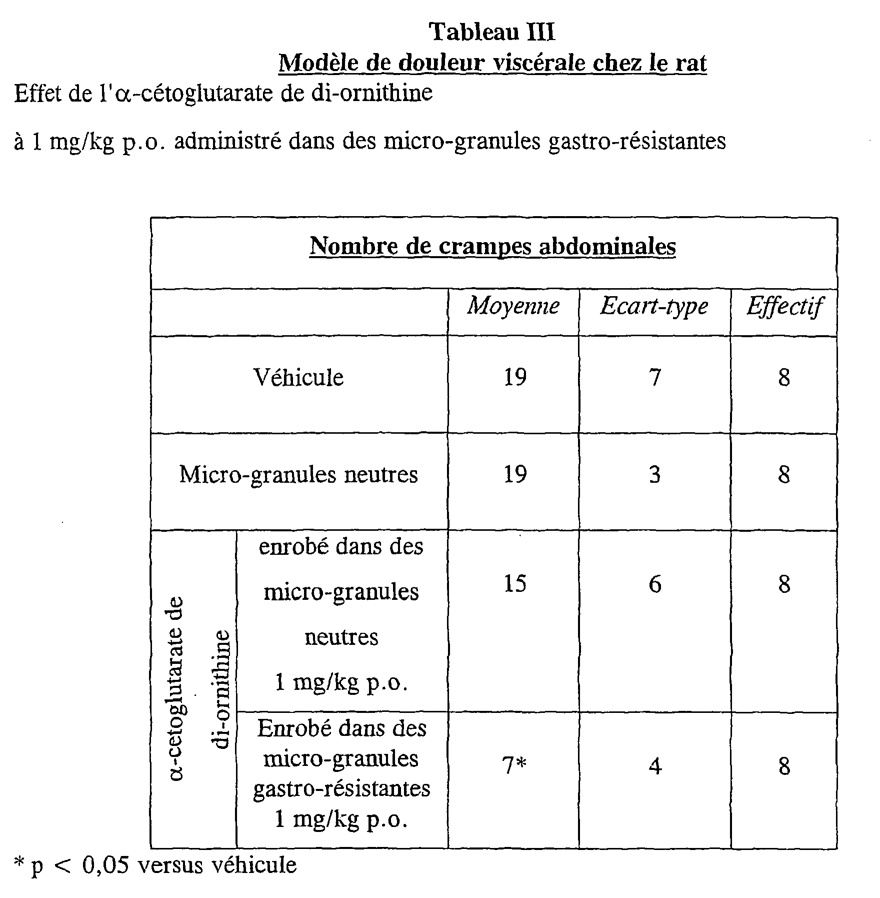

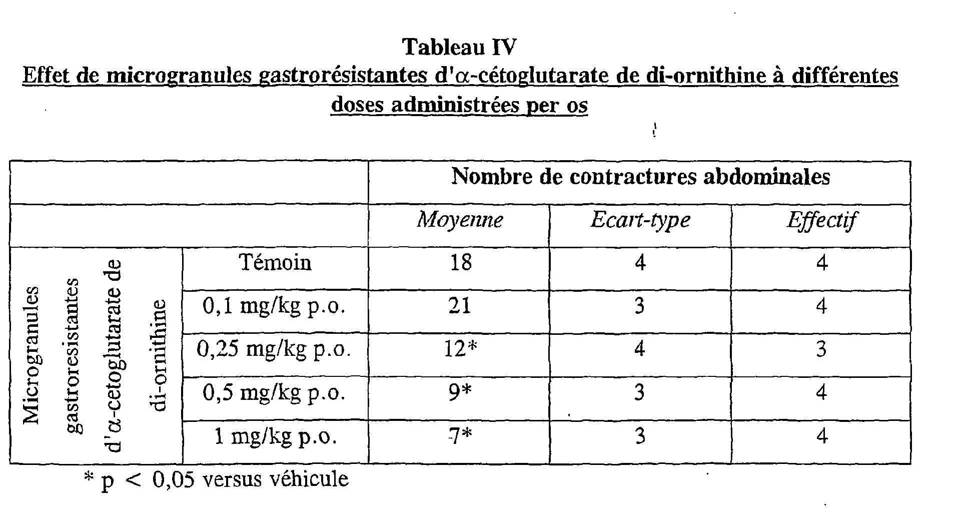

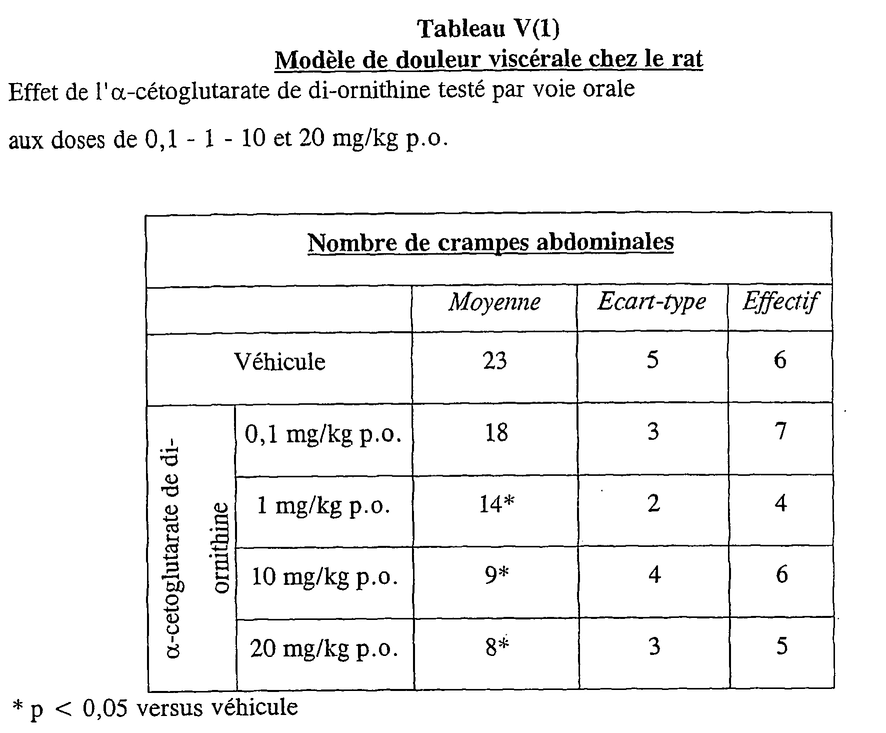

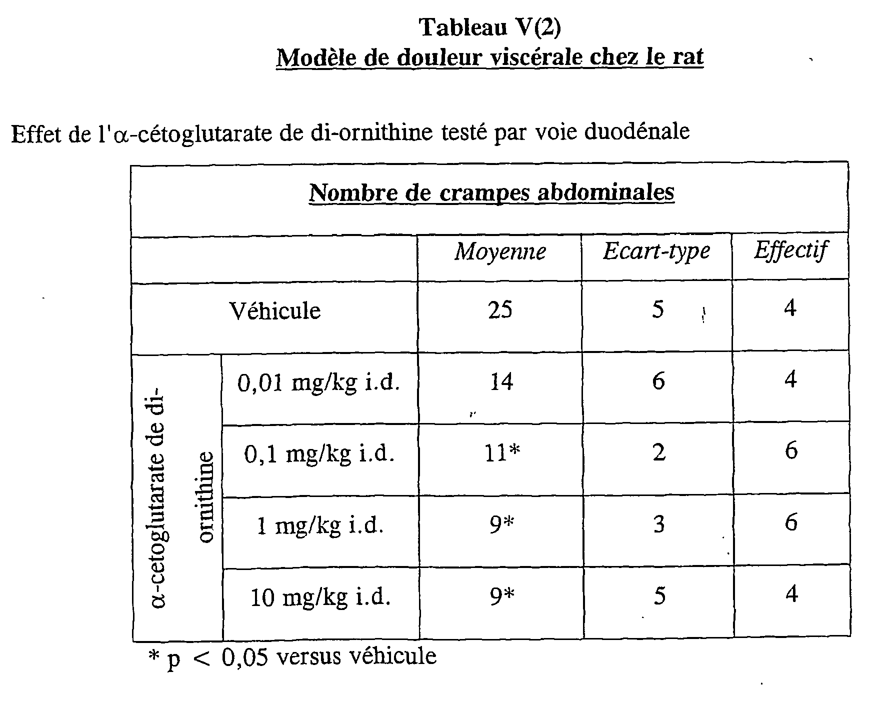

### Hypercatabolisme protéique, évolution pondérale et effet secondaire

L'irritation du côlon à l'acide acétique 4% provoque chez le rat une perte de poids d'environ 10 à 15 % . Ce stress chimique constitue un bon modèle d'hypercatabolisme pour rechercher l'activité nutritionnelle des molécules étudiées.

### Protocole.

L'évolution pondérale est enregistrée pendant 12 jours chez des rats femelles Wistar après avoir subi une irritation du côlon à l'acide acétique 4 %.
Des rats femelles Wistar d'environ 250g à jeun depuis 48h sont anesthésiées à l'halothane.
Le côlon est irrité à l'acide acétique dilué (1,5 ml d'une solution à 4 % sont introduits par voie rectale à 5cm de la marge anale).A leur réveil, les animaux répartis 5 par cage sont alimentés et abreuvés ad libitum. Deux jours plus tard, les animaux sont répartis en 3 groupes de rats de poids homogènes (sont exclus de l'étude ,les rats dont la perte de poids est inférieure à 5g ou supérieure à 30g).
Le produit à tester ou le véhicule (eau distillée) est administré par voie orale deux fois par jour, entre 8h et 10h le matin et entre 15h et 16h l'après midi.
L'évolution pondérale est enregistrée pendant 12 jours. Le poids des animaux est mesuré en grammes à l'aide d'une balance.
L'analyse statistique est effectuée à l'aide du test de Dunnett qui compare un même groupe d'animaux véhicule (50) à plusieurs groupes de rats (effectif compris entre 9 et 19 selon le groupe) ayant reçu l'une des molécules étudiées. Le seuil de signification est fixé à 5 % .
L'analyse statistique de l'effet secondaire diarrhée est effectuée à l'aide d'un test de χ² qui compare le groupe d'animaux ayant reçu la composition gastrorésistante au groupe ayant reçu
la composition non gastrorésistante sur les caractères diarrhée non diarrhée. Le seuil de signification est fixé à 1 %.

Les molécules sont testées par voie orale :
- en solution à pH contrôlé, à 1 et 3 mg / kg p.o.; Les produits sont solubilisés dans l'eau distillée, pour les solutions pH 6, en présence d'acide chlorhydrique 1 N pour les solutions pH 1. Le pH est contrôlé à l'aide d'un pH mètre. Le véhicule utilisé comme placebo se compose d'eau distillée.
- sous forme sèche dans une composition gastrorésistante versus composition non gastrorésistante, à 1,2 mg / kg p.o..

### Résultats

- Etude en solution :
   Dans le groupe de rats véhicule, le poids initial à J1 est égal à 204 ± 11g. Les animaux atteignent 248 ± 20g à J12 (N=50). A 312, le poids des animaux est significativement (p < 0.05) supérieur dans les groupes où les rats ont reçu de l'α-cétoglutarate de di-ornithine à 1 et 3mg/kg p.o. à pH=6.

résultats : tableau VI et figure 3

**Tableau VI**

| **Modèle de dénutrition chez le rat** | | | | | |
|---|---|---|---|---|---|
| Evolution pondérale (g) chez le rat Effet de l'α-cétoglutarate de di-ornithine pH6 versus pH1 testé aux doses de 1 et 3 mg/kg p.o. | | | | | |
| **Jours** | Véhicule | 1 mg (pH1) | 1 mg (pH6) | 3 mg (pH1) | 3 mg (pH6) |
| Effectif | 50 | 18 | 19 | 11 | 9 |
| J1 | 204 ± 11 | 204 ± 8 | 209 ± 10 | 205 ± 7 | 209 ± 10 |
| J2 | 202 ± 14 | 203 ± 10 | 213 ± 16 | 206 ± 11 | 213 ± 15 |
| J3 | 205 ± 17 | 207 ± 13 | 221 ± 19 | 207 ± 11 | 219 ± 19 |
| J4 | 210 ± 20 | 213 ± 17 | 229* ± 18 | 210 ± 14 | 226 ± 20 |
| J5 | 215 ± 22 | 219 ± 16 | 235* ± 18 | 215 ± 16 | 232* ± 18 |
| J8 | 232 ± 22 | 241 ± 11 | 255* ± 19 | 230 ± 21 | 247* ± 20 |
| J9 | 237 ± 21 | 246 ± 11 | 259* ± 19 | 236 ± 21 | 251* ± 19 |
| J10 | 241 ± 20 | 248 ± 13 | 262* ± 18 | 239 ± 20 | 258* ± 20 |
| J11 | 245 ± 20 | 253 ± 11 | 268* ± 19 | 240 ± 21 | 265* ± 21 |
| **J12** | 248 ± 20 | 256 ± 11 | 274* ± 18 | 244 ± 20 | 271* ± 21 |

| | | | | | |
|---|---|---|---|---|---|
| Poids moyen exprimé en grammes ± son écart type (SD) * p < 0,05 versus véhicule | | | | | |

### - Etude sous forme sèche :

Dans le groupe de rats ayant reçu la composition non gastrorésistante, le poids initial à J 1 est égal à 197 ± 7 g. Les animaux atteignent 230 ± 28 g à J 12, (N = 10). Dans le groupe de rats traités par la composition gastrorésistante, le poids des animaux est significativement supérieur dès J 5 (p < 0,05).
Les résultats sont décrits dans le tableau VII et figure 4.
L'effet secondaire diarrhées est significativement diminué dans le groupe traité par la forme gastrorésistante versus le groupe traité par la forme non gastrorésistante, au risque α = 1 % (résultats tableau VIII).

**Tableau VII**

| **Modèle de dénutrition chez le rat** | | |
|---|---|---|
| Evolution pondérale (g) chez le rat Effet de l'α-cétoglutarate de di-ornithine composition gastrorésistante versus composition non gastrorésistante, Testé à la dose de 1,2 mg/kg p.o. | | |
| **Jours** | **Non gastrorésistant 1,2 mg/kg** | **Gastrorésistant 1,2 mg/kg** |
| Effectif | 10 | 10 |
| J1 | 197 ± 7 | 198 ± 6 |
| J2 | 198 ± 11 | 201 ± 8 |
| J3 | 203 ± 14 | 211 ± 12 |
| J4 | 214 ± 17 | 220 ± 11 |
| J5 | 216 ± 20 | 229 ± 12* |
| J6 | 221 ± 17 | 233 ± 12* |
| J7 | 225 ± 21 | 237 ± 10* |
| J8 | 225 ± 26 | 239 ± 12* |
| J9 | 221 ± 32 | 242 ± 12* |
| J10 | 226 ± 29 | 245 ± 11* |
| J11 | 227 ± 31 | 250 ± 11* |
| J12 | 230 ± 28 | 253 ± 10* |

| | | |
|---|---|---|
| Poids moyen exprimé en grammes ± son écart-type (SD) * p < 0,05 versus véhicule | | |

**Tableau VIII**

| **Modèle de dénutrition chez le rat** **(Effet secondaire diarrhée)** | | |
|---|---|---|
| | **Non gastrorésistant** | **Gastrorésistant** |
| **Diarrhées** | 84 | 53 * |
| **Non diarrhée** | 36 | 67 |

| | | |
|---|---|---|
| *p< 0,01 | | |

### Légendes des figures

- figure 1 : Effet de microgranules gastrorésistantes d'α-cétoglutarate de di-ornithine sur le nombre de contractures abdominales.
- figure 2 : Effet de l'α-cétoglutarate de di-ornithine sur le nombre de crampes abdominales.
- figure 3 : Effet de l'α-cétoglutarate de di-ornithine en solution sur l'évolution pondérale chez le rat.
- figure 4 : Effet de l'α-cétoglutarate de di-ornithine en composition gastrorésistante sur l'évolution pondérale chez le rat.

## Revendications

1. Composition gastrorésistante comprenant au moins un composé de formule générale (I) suivante:
(X)ₙ₁,Y,(Z)n₂ (I)
dans laquelle:
- X et Z sont des acides aminés identiques ou différents, naturels ou non,
- Y est un cétoacide à chaîne linéaire non ramifiée,
- n₁ et n₂, indépendamment l'un de l'autre, représentent 0 ou 1, l'un au moins de n₁ et n₂ représentant 1, ledit composé se présentant sous forme de sel entre deux constituants X et Y, ou Y et Z, ou entre les trois constituants X, Y et Z.

2. Composition gastrorésistante selon la revendication 1, **caractérisée en ce qu'**elle comprend au moins un composé de formule (I), en association avec un véhicule gastrorésistant physiologiquement stable, ledit composé au sein de la composition, étant stable en milieu neutre et à pH inférieur à 6, jusqu'à un pH d'environ 1.

3. Composition gastrorésistante selon la revendication 1 ou 2, comprenant au moins un composé de formule générale (I) dans laquelle:
- n₁ et n₂, indépendamment l'un de l'autre, représentent 0 ou 1, l'un au moins de n₁ et n₂ représentant 1,
- X représente un aminoacide naturel, sous réserve que lorsque n₂ = 0 alors X représente un aminoacide basique tel que:
l'ornithine,
l'arginine,
la lysine,
ou, l'histidine,
- Y représente un cétoacide de formule (II) suivante:
R-CO-COOH (II)
dans laquelle R représente un groupe alkyle ou acide alcanoïque linéaire, d'environ 1 à environ 10 atomes de carbone, notamment un cétoacide de formule (II) dans laquelle lorsque
R représente:
- CH₃, ledit cétoacide est l'acide pyruvique,
- CH₂-CH₃, ledit cétoacide est l'acide α-cétobutyrique,
- (CH₂)₂-COOH, ledit cétoacide est l'acide α-cétoglutarique,
- (CH₂)₃-COOH, ledit cétoacide est l'acide α-cétoadipique,
Z représente un aminoacide naturel, notamment un aminoacide choisi parmi l'ornithine, l'arginine, la lysine, l'histidine, la proline, ou la glutamine.

4. Composition gastrorésistante selon l'une des revendications 1 à 3, **caractérisée en ce qu'**elle comprend au moins un composé choisi parmi ceux de formule (I) dans laquelle:
- n₁=1, et n₂=0 ou 1,
- X représente un acide aminé choisi parmi l'ornithine, la lysine, ou l'arginine,
- Y représente un cétoacide choisi parmi l'acide α-cétoglutarique, ou l'acide α- cétobutyrique,
- et, lorsque n₂ = 1, Z représente un acide aminé naturel, notamment l'ornithine, l'arginine, la proline, ou la glutamine.

5. Composition gastrorésistante selon l'une des revendications 1 à 4, **caractérisée en ce que** les composés sont choisis parmi ceux de formule (I) dans laquelle:
- n₁ = 1, et n₂ = 0,
- X représente l'ornithine et Y représente l'acide α-cétoglutarique, à savoir l'α-cétoglutarate de mono-ornithine,
- X représente l'ornithine et Y représente l'acide α-cétobutyrique, à savoir l'α-cétobutyrate de mono-ornithine,
- X représente l'arginine et Y représente l'acide α -cétobutyrique, à savoir l'α -cétobutyrate d'arginine,
- X représente la lysine et Y représente l'acide α-cétobutyrique, à savoir l'α-cétobutyrate de lysine,
- X représente l'histidine et Y représente l'acide α-cétobutyrique, à savoir l'α-cétobutyrate d'histidine.

6. Composition gastrorésistante selon l'une des revendications 1 à 4,
**caractérisée en ce que** les composés sont choisis parmi ceux de formule (I) dans laquelle:
- n₁=1, et n₂=1,
- X représente l'ornithine, Y représente l'acide α-cétoglutarique, et Z représente l'ornithine, à savoir l'α-cétoglutarate de di-ornithine,
- X représente l'arginine, Y représente l'acide α-cétoglutarique, et Z représente l'arginine, à savoir l'α-cétoglutarate de di-arginine,
- X représente l'ornithine, Y représente l'acide α-cétoglutarique, et Z représente la glutamine, à savoir l'α-cétoglutarate d'ornithine et de glutamine,
- X représente l'ornithine, Y représente l'acide α-cétoglutarique, et Z représente la proline, à savoir l'α-cétoglutarate d'ornithine et de proline.

7. Composition gastrorésistante selon l'une des revendications 1 à 4,
**caractérisée en ce que** le composé est l'α-cétoglutarate de di-ornithine.

8. Composition gastrorésistante selon l'une des revendications 1 à 7,
**caractérisée en ce que** les composés de formule (1) sont tels que la proportion en poids des différents constituants X, Y, et/ou Z, est de préférence comprise entre 0,8 et 1,2, de sorte que, la somme des proportions de chacun des constituants est égale à 2 dans un sel entre deux constituants, ou est égale à 3 dans un sel entre trois constituants.

9. Composition gastrorésistante selon l'une des revendications 1 à 8,
**caractérisée en ce que** les composés de formule (I) sont tels que les différents constituants X et Y, ou Y et Z, ou X, Y et Z sont dans un rapport équimolaire, c'est-à-dire que chacun des constituants est dans une proportion en poids de 1, de sorte que la somme des proportions de chacun des constituants est égale à 2 dans un sel entre deux constituants X et Y, ou Y et Z, ou est égale à 3 dans un sel entre les trois constituants X, Y et Z.

10. Composition gastrorésistante selon l'une des revendications 1 à 9,
**caractérisée en ce que** le véhicule gastrorésistant physiologiquement stable est choisi parmi:
- les microgranules gastrorésistantes,
- les microgranules pelliculées gastrorésistantes,
- les nanoparticules gastrorésistantes,
- les nanosphères gastrorésistantes,
- les microsphères gastrorésistantes,
- les microcapsules gastrorésistantes,
- les granulés gastrorésistants,
- les granulés pelliculés gastrorésistants,
- les liposomes gastrorésistants,
- les liposomes pelliculés gastrorésistants,
- les lyocs gastrorésistants,
- les lyocs pelliculés gastrorésistants,
- les pompes osmotiques dans un enrobage gastrorésistant,
- les gommes,
- les sphéroïdes gastrorésistants,
- les sphérules gastrorésistantes,
- les sphéroïdes pelliculés gastrorésistants,
- les sphérules pelliculées gastrorésistantes,
- les comprimés pelliculés gastrorésistants,
- les gélules pelliculées gastrorésistantes.

11. Composition gastrorésistante selon l'une des revendications 1 à 10, **caractérisée en ce qu'**elle se présente sous forme de comprimés, de gélules, de sachets, ou de granulés gastrorésistants.

12. Adjuvant de nutrition **caractérisé en ce qu'**il contient une composition gastrorésistante selon l'une des revendications 1 à 11, le cas échéant en association avec un véhicule acceptable en alimentation.

13. Adjuvant de nutrition selon la revendication 12, **caractérisé en ce qu'**il se présente sous une forme prête à diluer ou à disperser dans un solvant aqueux.

14. Adjuvant de nutrition selon la revendication 12 ou la revendication 13,
**caractérisé en ce qu'**il se présente sous forme de prise unitaire contenant environ 25 mg à environ 20 g d'au moins un composé de la composition, pour une posologie journalière comprise entre environ 25 mg à environ 40 g.

15. Composition pharmaceutique **caractérisée en ce qu'**elle contient une composition gastrorésistante selon l'une des revendications 1 à 11, le cas échéant en association avec un véhicule pharmaceutiquement acceptable.

16. Composition pharmaceutique selon la revendication 15, **caractérisée en ce qu'**elle se présente sous une forme administrable par voie orale, ou par voie entérale, notamment sous une forme sèche à diluer ou à disperser dans un solvant aqueux lorsqu'elle est sous une forme administrable par voie orale.

17. Composition pharmaceutique selon la revendication 15 ou la revendication 16, **caractérisée en ce qu'**elle est administrable par voie orale sous forme de prise unitaire contenant environ 25 mg à environ 20 g de composé de la composition, pour une posologie journalière en principe actif comprise entre environ 1 mg/kg/jour à 1 g/kg/jour.

18. Utilisation de compositions gastrorésistantes comprenant au moins un composé de formule générale (Ibis) suivante:
(X)ₙ₁,Y,(Z)ₙ₂ (Ibis)
dans laquelle:
- X et Z sont des acides aminés identiques ou différents, naturels ou non,
- Y est un cétoacide à chaîne ramifiée,
- n₁ et n₂, indépendamment l'un de l'autre, représentent 0 ou 1, l'un au moins de n₁ et n₂ représentant 1, ledit composé se présentant sous forme de sel entre deux constituants X et Y, ou Y et Z, ou entre les trois constituants X, Y et Z,
pour la préparation d'un adjuvant de nutrition, ou d'un médicament, destinés au traitement des états de dénutrition, **ou au traitement symptomatique des douleurs associées aux pathologies du tube digestif, de la vessie et des voies biliaires**.

19. Utilisation de compositions gastrorésistantes selon la revendication 18, pour la préparation d'un adjuvant de nutrition ou d'un médicament destiné au traitement des états de dénutrition chez:
- les sujets dénutris,
- les sujets anorexiques,
- les patients atteints de la gastroparésie,
- les sujets dont le transit digestif est ralenti,
- les sujets souffrant de malabsorption digestive,
- les patients atteints de la maladie d'Alzheimer,
- les personnes en situation d'hypercatabolisme, telsque:
■ les personnes atteintes d'insuffisance respiratoire,
■ les personnes souffrant d'escarres,
■ les patients brûlés,
■ les patients cancéreux,
■ les patients atteints du SIDA,
■ les patients post-opérés,
■ les personnes en cours de cicatrisation de la muqueuse intestinale,
■ les patients polytraumatisés,
■ les patients insuffisants cardiaques,
ou dans le cadre de la cicatrisation et de la stimulation de l'hormone de croissance, ou de l'insuline.

20. Utilisation de compositions gastrorésistantes selon la revendication 18, pour la préparation d'un médicament destiné au traitement symptomatique des douleurs:
■ des troubles du transit et de l'inconfort intestinal liés aux troubles fonctionnels intestinaux (dyspepsies, syndrome de l'intestin irritable, côlon irritable,...)
■ dans la rectocolite hémorragique,
■ dans la maladie de Crohn,
■ dans l'ulcère gastrique et duodénal,
■ dans la gastrite chronique,
■ dans le cancer colorectal ou gastrique,
■ dans la gastroentérite et la grippe intestinale,
■ liées à une pseudo-obstruction intestinale ou colique,
■ dans l'iléite radique,
■ post-opératoires digestives ou viscérales,
■ dans la diarrhée, spasmes, constipations, mégacôlon, le mégarectum,
■ des spasmes de la vessie,
■ de la parésie vésicale.

21. Utilisation de compositions gastrorésistantes selon l'une des revendications 18 à 20, comprenant au moins un composé de formule générale (Ibis) dans laquelle:
- n₁ et n₂, indépendamment l'un de l'autre, représentent 0 ou 1, l'un au moins de n₁ et n₂ représentant 1,
- X représente un aminoacide naturel, sous réserve que lorsque n₂ = 0 alors X représente un aminoacide basique tel que:
l'ornithine,
l'arginine,
la lysine,
ou l'histidine,
- Y représente un cétoacide de formule (II) suivante:
R-CO-COOH (II)
dans laquelle R représente un groupe alkyle ou acide alcanoïque ramifié, d'environ 1 à environ 10 atomes de carbone, notamment un cétoacide de formule (II) dans laquelle lorsque R représente:
- CH(CH₃)₂, ledit cétoacide est l'acide α-cétoisovalérique,
- CH(CH₃)-CH₂-CH₃, ledit cétoacide est l'acide α-céto β-méthylvalérique,
- CH₂-CH(CH₃)₂, ledit cétoacide est l'acide α-cétoisocaproïque.

22. Utilisation de compositions gastrorésistantes selon l'une des revendications 18 à 21 comprenant au moins un composé de formule générale (Ibis) dans laquelle:
- X représente l'arginine et Y représente l'acide α-céto-isocaproïque, à savoir l'α-céto-isocaproate d'arginine,
- X représente l'ornithine et Y représente l'acide α-céto-isocaproïque, à savoir l'α-céto-isocaproate d'ornithine,
- X représente l'ornithine et Y représente l'acide α-céto-β-méthylvalérique, à savoir l'α-céto-β-méthylvalérate d'ornithine,
- X représente l'arginine et Y représente l'acide α-céto-β-méthylvalérique, à savoir l'α-céto-β-méthylvalérate d'arginine,
- X représente l'arginine et Y représente l'acide α-céto-isovalérique, à savoir l'α-céto-isovalérate d'arginine,
- X représente l'ornithine et Y représente l'acide α-céto-isovalérique, à savoir l'α-céto-isovalérate d'ornithine.

## Claims

1. A gastroresistant composition comprising at least one compound of the following formula (I):
(X)n₁,Y,(Z)n₂ (I)
wherein:
- X and Z are identical or different, natural or non-natural amino acids,
- Y is an keto acid with a linear, unbranched chain,
- n₁ and n₂ independently represent 0 or 1, at least one of n₁ and n₂ representing 1,
the compound being presented in the form of a salt between two constituents X and Y or Y and Z or between the three constituents X, Y and Z.

2. The gastroresistant composition according to claim 1, **characterised in that** it comprises at least one compound of the formula (I) in association with a gastroresistant physiologically stable vehicle, said compound in the composition being stable in a neutral medium and at a pH of less than 6 to a pH of about 1.

3. The gastroresistant composition according to claim 1 or 2, comprising at least one compound of the general formula (I) wherein:
- n₁ and n₂ independently represent 0 or 1, at least one of n₁ and n₂ representing 1,
- X represents a natural amino acid, with the proviso that, when n₂ is 0, X represents a basic amino acid such as:
ornithine,
arginine,
lysine, or
histidine,
- Y represents a keto acid of the following formula (II):
R-CO-COOH (II)
wherein R represents an alkyl or linear alkanoic acid group having about 1 to about 10 carbon atoms, especially a keto acid of the formula (II) wherein, when R represents:
- CH₃, said keto acid is pyruvic acid,
- CH₂-CH₃, said keto acid is α-keto butyric aid,
- (CH₂)₂-COOH, said keto acid is α-keto glutaric acid
- (CH₂)₃-COOH, said keto acid is α-keto adipic acid,
Z represents a natural amino acid, especially an amino acid selected from ornithine, arginine, lysine, histidine, proline, or glutamine.

4. The gastroresistant composition according to any of the claims 1 to 3, **characterised in that** it comprises at least one compound selected among those of the formula (I) wherein:
- n₁ = 1 and n₂ = 0 or 1,
- X represents an amino acid selected among ornithine, lysine, or arginine,
- Y represents a keto acid selected among α-keto glutaric acid and α-keto butyric acid,
- and, when n₂ = 1, Z represents a natural amino acid, especially ornithine, arginine, proline, or glutamine.

5. The gastroresistant composition according to any of the claims 1 to 4, **characterised in that** the compounds are selected among those of the formula (I) wherein:
- n₁ = 1, and n₂ = 0,
- X represents ornithine and Y represents α-keto glutaric acid, i.e. the α-keto glutarate of monoornithine,
- X represents ornithine and Y represents α-keto butyric acid, i.e. the α-keto butyrate of monoornithine,
- X represents arginine and Y represents α-keto butyric acid, i.e. the α-keto butyrate of arginine,
- X represents lysine and Y represents α-keto butyric acid, i.e. the α-keto butyrate of lysine,
- X represents histidine and Y represents α-keto butyric acid, i.e. the α-keto butyrate of histidine.

6. The gastroresistant composition according to any of the claims 1 to 4, **characterised in that** the compounds are selected among those of the formula (I) wherein:
- n₁ = 1 and n₂ = 1,
- X represents ornithine, Y represents α-keto glutaric acid, and Z represents ornithine, i.e. the α-keto glutarate of diornithine,
- X represents arginine, Y represents α-keto glutaric acid, and Z represents arginine, i.e. the α-keto glutarate of diarginine,
- X represents ornithine, Y represents α-keto glutaric acid, and Z represents glutamine, i.e. the α-keto glutarate of ornithine and glutamine,
- X represents ornithine, Y represents α-keto glutaric acid, and Z represents proline, i.e. the α-keto glutarate of ornithine and proline.

7. The gastroresistant composition according to any of the claims 1 to 4, **characterised in that** the compound is the α-keto glutarate of diornithine.

8. The gastroresistant composition according to any of the claims 1 to 7, **characterised in that** the compounds of the formula (I) are such that the weight ratio of the different constituents X, Y and/or Z is preferably between 0,8 and 1,2 so that the sum of the proportionate amount of each of the constituents in a salt is 2 between two constituents or 3 between three constituents in a salt.

9. The gastroresistant composition according to any of the claims 1 to 8, **characterised in that** the compounds of the formula (I) are such that the different constituents X and Y, or Y and Z, or X, Y and Z are present in an equimolar ratio, i.e. that each of the constituents is present in a weight ratio of 1 so that the sum of the proportionate amount of each of the constituents in a salt is 2 between two constituents X and Y or Y and Z in a salt or is 3 between the three constituents X, Y and Z in a salt.

10. The gastroresistant composition according to any of the claims 1 to 9, **characterised in that** the gastroresistant physiologically stable vehicle is selected among:
- gastroresistant microgranules,
- gastroresistant coated microgranules,
- gastroresistant nanoparticles,
- gastroresistant nanospheres
- gastroresistant microspheres,
- gastroresistant microcapsules,
- gastroresistant granules,
- gastroresistant coated granules,
- gastroresistant liposomes,
- coated gastroresistant liposomes,
- gastroresistant lyocs,
- coated gastroresistant lyocs,
- osmotic pumps having a gastroresistant coating,
- gums,
- gastroresistant spheroids,
- gastroresistant spheres,
- coated gastroresistant spheroids,
- coated gastroresistant spheres,
- coated gastroresistant tablets,
- coated gastroresistant gelatine capsules.

11. The gastroresistant composition according to any of the claims 1 to 10, **characterised in that** it is presented in the form of gastroresistant tablets, gelatine capsules, sachets or granules.

12. A food additive **characterised in that** it contains a gastroresistant composition according to any of the claims1 to 11, optionally in association with a vehicle compatible with the diet.

13. The food additive according to claim 12, **characterised in that** it is presented in a form ready for dilution or dispersion in an aqueous solvent.

14. The food additive according to claim 12 or claim13, **characterised in that** it is presented in a single dose form containing about 25 mg to about 20 g of at least one compound of the composition for daily posology which comprises between about 25 mg and about 40 g.

15. A pharmaceutical composition **characterised in that** it contains a gastroresistant composition according to any of the claims 1 to 11, optionally in association with a pharmaceutically acceptable vehicle.

16. The pharmaceutical composition according to claim 15, **characterised in that** it is presented in a form that may be administered by the oral route or enteral route, especially in a dry form to be diluted or dispersed in an aqueous solvent when in a form to be administered by the oral route.

17. The pharmaceutical composition according to claim 15 or claim 16, **characterised in that** it may be administered by the oral route in the form of a single dose containing about 25 mg to about 20 g of the compound of the composition for daily posology as an active ingredient at between about 1 mg/kg/day and 1 g/kg/day.

18. Use of gastroresistant compositions comprising at least one compound of the following formula (Ibis):
(X)n₁, Y, (Z)n₂ (Ibis)
wherein:
- X and Z are identical or different, natural or non-natural amino acids,
- Y is an keto acid with a branched chain,
- n₁ and n₂ independently represent 0 or 1, at least one of n₁ and n₂ representing 1, said compound being presented in the form of a salt between two constituents X and Y or Y and Z or between the three constituents X, Y and Z,
for the preparation of a food additive or a medicament designed for the treatment of conditions of nutrition deficiencies or the symptomatic treatment of pain associated with pathologies of the intestinal tract, the bladder and the bile ducts.

19. The use of gastroresistant compositions according to claim 18 for the preparation of a food additive or a medicament designed for the treatment of conditions of nutrition deficiency in
- patients with nutritional deficiency,
- anorexic patients,
- patients suffering from gastroparesis,
- persons where the passage through the intestinal tract is slowed down,
- persons suffering from digestive malabsorption,
- persons suffering from Alzheimer's disease,
- persons with hypercatabolism such as
■ persons suffering from respiratory insufficiency,
■ persons suffering from decubital ulcers,
■ patients with burns,
■ cancer patients,
■ AIDS patients,
■ patients after surgery,
■ patients during wound healing of the intestinal mucous membrane,
■ patients with multiple trauma
■ patients with cardiac insufficiency
or in connection with wound healing and the stimulation of the growth hormone or insulin.

20. The use of gastroresistant compositions according to claim 18 for the preparation of a medicament designed for the symptomatic treatment of pain:
• in case of disorders of the intestinal passage and intestinal discomfort caused by functional intestinal disorders (dyspepsia, irritabile bowel syndrome, irritable colon, ...)
• in case of haemorrhagic proctocolitis,
• in case of Morbus Crohn,
• in case of *Ulcus ventriculi* and *Ulcus duodeni,*
• in case of chronic gastritis,
• in case of colorectal or gastric cancer,
• in case of gastroenteritis and abdominal influenza, caused by an intestinal pseudoobstruction or colic,
• in case of radioileitis,
• after surgery to the intestinal tract or after visceral surgery,
• in case of diarrhoea, spasms, constipations, megacolon, megarectum,
• spasms of the bladder
• vesical paresis.

21. The use of gastroresistant compositions according to any of the claims 18 to 20 comprising at least one compound of the general formula (Ibis), wherein:
- n₁ and n₂ are independently 0 or 1, at least one of n₁ and n₂ being 1,
- X is a natural amino acid with the proviso that, when n₂ = 0, X is a basic amino acid such as
ornithine,
arginine,
lysine, or
histidine,
- Y is a keto acid of the following formula (II)
R-CO-COOH (II)
wherein R represents an alkyl or branched alkanoic acid group having about 1 to about 10 carbon atoms, especially a keto acid of the formula (II) wherein, when R represents:
- CH(CH₃)₂, said keto acid is the α-keto isovaleric acid,
- CH(CH₃)-CH₂-CH₃, said keto acid is the α-keto-β-methyl valeric acid,
- CH₂-CH(CH₃)₂, said keto acid is the α-keto isocaproic acid.

22. The use of gastroresistant compositions according to any of the claims 18 to 21 comprising at least one compound of the general formula (Ibis) wherein
- X represents arginine and Y represents the α-keto isocaproic acid, i.e. the α-keto isocaproate of arginine,
- X represents ornithine and Y represents the the α-keto isocaproic acid, i.e. the α-keto isocaproate of ornithine,
- X represents ornithine and Y represents the α-keto-β-methyl valeric acid, i.e. the α-keto-β-methyl va-lerate of ornithine,
- X represents arginine and Y represents the α-keto-β-methyl valeric acid, i.e. the α-keto-β-methyl va-lerate of arginine,
- X represents arginine and Y represents the α-keto isovaleric acid, i.e. the α-keto isovalerate of arginine,
- X represents ornithine and Y represents the α-keto isovaleric acid, i.e. the α-keto isovalerate of ornithine.

## Patentansprüche

1. Magensaftresistente Zusammensetzung, die wenigstens eine Verbindung der folgenden allgemeinen Formel (I) umfasst:
(X)ₙ₁Y,(Z)ₙ₂ (I)
in der:
- X und Z identische oder unterschiedliche, natürliche oder nicht-natürliche Aminosäuren sind,
- Y eine Ketosäure mit linearer nichtverzweigter Kette ist,
- n₁ und n₂ unabhängig voneinander 0 oder 1 darstellen,
wobei wenigstens eines von n₁ und n₂ 1 darstellt,
wobei sich die Verbindung in Salzform zwischen zwei Bestandteilen X und Y oder Y und Z oder zwischen den drei Bestandteilen X, Y und Z präsentiert.

2. Magensaftresistente Zusammensetzung nach Anspruch 1,
**dadurch gekennzeichnet, dass** sie wenigstens eine Verbindung der Formel (I) in Assoziation mit einem magensaftresistenten physiologisch stabilen Vehikel umfasst, wobei die Verbindung in der Zusammensetzung in neutralem Medium und bei einem pH unter 6 bis zu einem pH von etwa 1 stabil ist.

3. Magensaftresistente Zusammensetzung nach Anspruch 1 oder 2, die wenigstens eine Verbindung der allgemeinen Formel (I) umfasst, in der:
- n₁ und n₂ unabhängig voneinander 0 oder 1 darstellen,
wobei wenigstens eines von n₁ und n₂ 1 darstellt,
- X eine natürliche Aminosäure darstellt, mit der Maßgabe, dass, wenn n₂ gleich 0 ist, X dann eine basische Aminosäure wie:
Ornithin,
Arginin,
Lysin oder
Histidin darstellt,
- Y eine Ketosäure der folgenden Formel (II) darstellt:
R-CO-COOH (II)
in der R eine Alkyl- oder lineare Alkansäuregruppe mit etwa 1 bis etwa 10 Kohlenstoffatomen darstellt, insbesondere eine Ketosäure der Formel (II) darstellt, in der, wenn R
- CH₃ darstellt, die Ketosäure Brenztraubensäure ist,
- CH₂-CH₃ darstellt, die Ketosäure α-Ketobuttersäure ist,
- (CH₂)₂-COOH darstellt, die Ketosäure α-Ketoglutarsäure ist,
- (CH₂)₃-COOH darstellt, die Ketosäure α-Ketoadipinsäure ist,
Z eine natürliche Aminsäure darstellt, insbesondere eine Aminosäure, die aus Ornithin, Arginin, Lysin, Histidin, Prolin und Glutamin ausgewählt ist.

4. Magensaftresistente Zusammensetzung nach einem der Ansprüche 1 bis 3, **dadurch gekennzeichnet, dass** sie wenigstens eine Verbindung umfasst, die unter denen der Formel (I) ausgewählt ist, in der:
- n₁ gleich 1 und n₂ 0 oder 1 ist,
- X eine Aminosäure darstellt, ausgewählt aus Ornithin, Lysin und Arginin,
- Y eine Ketosäure darstellt, ausgewählt aus α-Ketoglutarsäure und α-Ketobuttersäure,
- und wenn n₂ = 1, Z eine natürliche Aminosäure darstellt, insbesondere Ornithin, Arginin, Prolin oder Glutamin.

5. Magensaftresistente Zusammensetzung nach einem der Ansprüche 1 bis 4, **dadurch gekennzeichnet, dass** die Verbindungen unter denen der Formel (I) ausgewählt sind, in der:
- n₁ = 1 und n₂ = 0,
- X Ornithin darstellt und Y α-Ketoglutarsäure darstellt, das heißt das α-Ketoglutarat von Monoornithin,
- X Ornithin darstellt und Y α-Ketobuttersäure darstellt, das heißt das α-Ketobutyrat von Monoornithin,
- X Arginin darstellt und Y α-Ketobuttersäure darstellt, das heißt das α-Ketobutyrat von Arginin,
- X Lysin darstellt und Y α-Ketobuttersäure darstellt, das heißt das α-Ketobutyrat von Lysin,
- X Histidin darstellt und Y α-Ketobuttersäure darstellt, das heißt das α-Ketobutyrat von Histidin.

6. Magensaftresistente Zusammensetzung nach einem der Ansprüche 1 bis 4, **dadurch gekennzeichnet, dass** die Verbindungen unter denen der Formel (I) ausgewählt sind, in der:
- n₁ = 1 und n₂ = 1,
- X Ornithin darstellt, Y α-Ketoglutarsäure darstellt und Z Ornithin darstellt, das heißt das α-Ketoglutarat von Diornithin,
- X Arginin darstellt, Y α-Ketoglutarsäure darstellt und Z Arginin darstellt, das heißt das α-Ketoglutarat von Diarginin,
- X Ornithin darstellt, Y α-Ketoglutarsäure darstellt und Z Glutamin darstellt, das heißt das α-Ketoglutarat von Ornithin und Glutamin,
- X Ornithin darstellt, Y α-Ketoglutarsäure darstellt und Z Prolin darstellt, das heißt das α-Ketoglutarat von Ornithin und Prolin.

7. Magensaftresistente Zusammensetzung nach einem der Ansprüche 1 bis 4, **dadurch gekennzeichnet, dass** die Verbindung das α-Ketoglutarat von Diornithin ist.

8. Magensaftresistente Zusammensetzung nach einem der Ansprüche 1 bis 7, **dadurch gekennzeichnet, dass** die Verbindungen der Formel (I) so sind, dass das Gewichtsverhältnis der verschiedenen Bestandteile X, Y und/oder Z vorzugsweise zwischen 0,8 und 1,2 liegt, derart, dass die Summe der Verhältnismengen jedes der Bestandteile in einem Salz zwischen 2 Bestandteilen gleich zwei ist oder in einem Salz zwischen 3 Bestandteilen gleich drei ist.

9. Magensaftresistente Zusammensetzung nach einem der Ansprüche 1 bis 8, **dadurch gekennzeichnet, dass** die Verbindungen der Formel (I) so sind, dass die verschiedenen Bestandteile X und Y oder Y und Z oder X, Y und Z in einem äquimolaren Verhältnis sind, das heißt, dass jeder der Bestandteile in einem Gewichtsverhältnis von 1 ist, derart, dass die Summe der Verhältnisanteile jedes der Bestandteile in einem Salz zwischen zwei Bestandteilen X und Y oder Y und Z gleich zwei ist oder in einem Salz zwischen den drei Bestandteilen X, Y und Z gleich drei ist.

10. Magensaftresistente Zusammensetzung nach einem der Ansprüche 1 bis 9, **dadurch gekennzeichnet, dass** das magensaftresistente physiologisch stabile Vehikel ausgewählt ist aus:
- den magensaftresistenten Mikrokörnchen,
- den magensaftresistenten beschichteten Mikrokörnchen,
- den magensaftresistenten Nanopartikeln,
- den magensaftresistenten Nanosphären,
- den magensaftresistenten Mikrosphären,
- den magensaftresistenten Mikrokapseln,
- den magensaftresistenten Granulaten,
- den beschichteten magensaftresistenten Granulaten,
- den magensaftresistenten Liposomen,
- den beschichteten magensaftresistenten Liposomen,
- den magensaftresistenten Lyocs,
- den beschichteten magensaftresistenten Lyocs,
- den osmotischen Pumpen in einer magensaftresistenten Umhüllung,
- den Gummen,
- den magensaftresistenten Sphäroiden,
- den magensaftresistenten Kügelchen,
- den beschichteten magensaftresistenten Sphäroiden,
- den beschichteten magensaftresistenten Kügelchen,
- den beschichteten magensaftresistenten Tabletten,
- den beschichteten magensaftresistenten Gelatinekapseln.

11. Magensaftresistente Zusammensetzung nach einem der Ansprüche 1 bis 10, **dadurch gekennzeichnet, dass** sie sich in Form von magensaftresistenten Tabletten, Gelatinekapseln, Sachets oder Granulat präsentiert.

12. Nahrungsadditiv, **dadurch gekennzeichnet, dass** es eine magensaftresistente Zusammensetzung nach einem der Ansprüche 1 bis 11, gegebenenfalls in Assoziation mit einem mit der Ernährung verträglichen Vehikel enthält.

13. Nahrungsadditiv nach Anspruch 12, **dadurch gekennzeichnet, dass** es sich in einer Form präsentiert, die bereit ist, um in einem wässrigen Lösungsmittel verdünnt oder dispergiert zu werden.

14. Nahrungsadditiv nach Anspruch 12 oder Anspruch 13, **dadurch gekennzeichnet, dass** es sich in einer Einzeldosisform präsentiert, die etwa 25 mg bis etwa 20 g wenigstens einer Verbindung der Zusammensetzung enthält, für eine tägliche Posologie, die zwischen etwa 25 mg und etwa 40 g umfasst.

15. Pharmazeutische Zusammensetzung, **dadurch gekennzeichnet, dass** sie eine magensaftresistente Zusammensetzung nach einem der Ansprüche 1 bis 11 gegebenenfalls in Assoziation mit einem pharmazeutisch verträglichen Vehikel enthält.

16. Pharmazeutische Zusammensetzung nach Anspruch 15, **dadurch gekennzeichnet, dass** sie sich in einer Form präsentiert, die auf oralem Wege oder auf enteralem Wege verabreichbar ist, insbesondere in trockener Form, die in einem wässrigen Lösungsmittel zu verdünnen oder zu dispergieren ist, wenn sie in einer auf oralem Weg verabreichbaren Form ist.

17. Pharmazeutische Zusammensetzung nach Anspruch 15 oder Anspruch 16, **dadurch gekennzeichnet, dass** sie auf oralem Weg in Form einer Einzeldosis verabreichbar ist, die etwa 25 mg bis etwa 20 g Verbindung der Zusammensetzung enthält, und zwar für eine tägliche Posologie als Wirkstoff mit zwischen etwa 1 mg/kg/Tag bis 1 g/kg/Tag.

18. Verwendung von magensaftresistenten Zusammensetzungen, die wenigstens eine Verbindung der folgenden allgemeinen Formel (Ibis) umfassen:
**(X)ₙ₁,Y,(Z)ₙ₂ (Ibis)**
in der:
- X und Z identische oder unterschiedliche, natürliche oder nicht-natürliche Aminosäuren sind,
- Y eine Ketosäure mit verzweigter Kette ist,
- n₁ und n₂ unabhängig voneinander 0 oder 1 darstellen,
wobei wenigstens eines von n₁ und n₂ 1 darstellt, wobei sich die Verbindung in Form eines Salzes zwischen zwei Bestandteilen X und Y oder Y und Z oder zwischen den drei Bestandteilen X, Y und Z präsentiert,
für die Herstellung eines Nahrungsadditivs oder eines Medikaments, die zur Behandlung von Mangelernährungszuständen oder zur symptomatischen Behandlung von Schmerzen, assoziiert mit Pathologien des Verdauungstrakts, der Blase und der Gallenwege, bestimmt sind.

19. Verwendung von magensaftresistenten Zusammensetzungen nach Anspruch 18 für die Herstellung eines Nahrungsadditivs oder eines Medikaments, bestimmt zur Behandlung von Mangelernährungszuständen bei:
- mangelernährten Patienten,
- anorexischen Personen,
- Patienten, die an Gastroparese erkrankt sind,
- Personen, bei denen die Passage durch den Verdauungstrakt verlangsamt ist,
- Personen, die an einer Verdauungsmalabsorption leiden,
- Patienten, die an Alzheimerscher Krankheit erkrankt sind,
- Personen mit Hyperkatabolismus wie:
■ Personen, die an respiratorischer Insuffizienz leiden,
■ Personen, die an Dekubitalgeschwüren leiden,
■ Patienten mit Verbrennungen,
■ Krebspatienten,
■ Patienten, die an AIDS leiden,
■ Patienten nach einer Operation,
■ Personen während einer Wundheilung der intestinalen Schleimhaut,
■ mehrfach traumatisierten Patienten,
■ Patienten mit Herzinsuffizienz
oder im Rahmen der Wundheilung und der Stimulation des Wachstumshormons oder von Insulin.

20. Verwendung von magensaftresistenten Zusammensetzungen gemäß Anspruch 18 für die Herstellung eines Medikaments, bestimmt zur symptomatischen Behandlung von Schmerzen:
■ bei Störungen der Darmpassage und intestinalem Unbehagen, bedingt durch funktionelle intestinale Störungen (Dyspepsien, Reizdarmsyndrom, Reizcolon, ...)
■ bei hämorrhagischer Proktokolitis,
■ bei Morbus Crohn,
■ bei Ulcus ventriculi und Ulcus duodeni,
■ bei chronischer Gastritis,
■ bei kolorektalem oder gastrischem Krebs,
■ bei Gastroenteritis und Darmgrippe,
■ bedingt durch eine intestinale Pseudoobstruktion oder Kolik,
■ bei Radioileitis,
■ nach Operationen des Verdauungstrakts oder nach Viszeraloperationen,
■ bei Diarrhöe, Spasmen, Konstipationen, Megacolon, Megarectum,
■ Spasmen der Blase,
■ der vesikalen Parese.

21. Verwendung von magensaftresistenten Zusammensetzungen nach einem der Ansprüche 18 bis 20, die wenigstens eine Verbindung der allgemeinen Formel (Ibis) umfassen, in der:
- n₁ und n₂ unabhängig voneinander 0 oder 1 darstellen,
wobei wenigstens eines von n₁ und n₂ 1 darstellt,
- X eine natürliche Aminosäure darstellt, mit der Maßgabe, dass, wenn n₂ = 0 ist, dann X eine basische Aminosäure wie:
Ornithin,
Arginin,
Lysin oder
Histidin darstellt,
- Y eine Ketosäure der folgenden Formel (II) darstellt:
R-CO-COOH (II)
in der R eine Alkyl- oder verzweigte Alkansäuregruppe mit etwa 1 bis etwa 10 Kohlenstoffatomen darstellt, insbesondere eine Ketosäure der Formel (II), in der, wenn R darstellt:
- CH(CH₃)₂, die Ketosäure die α-Ketoisovaleriansäure ist,
- CH(CH₃)-CH₂-CH₃, die Ketosäure die α-Keto-β-Methylvaleriansäure ist,
- CH₂-CH(CH₃)₂, die Ketosäure die α-Ketoisocapronsäure ist.

22. Verwendung von magensaftresistenten Zusammensetzungen nach einem der Ansprüche 18 bis 21, die wenigstens eine Verbindung der allgemeinen Formel (Ibis) umfassen, in der:
- X Arginin darstellt und Y die α-Ketoisocapronsäure darstellt, das heißt das α-Ketoisocapronat von Arginin,
- X Ornithin darstellt und Y die α-Ketoisocapronsäure darstellt, das heißt das α-Ketoisocapronat von Ornithin,
- X Ornithin darstellt und Y die α-Keto-β-Methylvaleriansäure darstellt, das heißt das α-Keto-β-Methylvalerat von Ornithin,
- X Arginin darstellt und Y die α-Keto-β-Methylvaleriansäure darstellt, das heißt das α-Keto-β-Methylvalerat von Arginin,
- X Arginin darstellt und Y die α-Ketoisovaleriansäure darstellt, das heißt das α-Ketoisovalerat von Arginin,
- X Ornithin darstellt und Y die α-Ketoisovaleriansäure darstellt, das heißt das α-Ketoisovalerat von Ornithin.
